(19) Europäisches Patentamt / European Patent Office / Office européen des brevets

(11) **EP 3 489 341 A1**

(12) **EUROPEAN PATENT APPLICATION**
published in accordance with Art. 153(4) EPC

(43) Date of publication:
**29.05.2019 Bulletin 2019/22**

(21) Application number: **17831137.9**

(22) Date of filing: **21.07.2017**

(51) Int Cl.:
***C12M 1/00** (2006.01)* ***C12N 1/00** (2006.01)*

(86) International application number:
**PCT/JP2017/026453**

(87) International publication number:
**WO 2018/016621 (25.01.2018 Gazette 2018/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.07.2016 JP 2016144929**

(71) Applicants:
- **Nissan Chemical Corporation**
  **Tokyo 103-6119 (JP)**
- **Kohjin Bio Co., Ltd.**
  **Sakado-shi, Saitama 350-0214 (JP)**

(72) Inventors:
- **TAKEUCHI, Yoshio**
  **Sakado-shi**
  **Saitama 350-0214 (JP)**
- **KANAKI, Tatsuro**
  **Shiraoka-shi**
  **Saitama 349-0294 (JP)**
- **SARUHASHI, Koichiro**
  **Funabashi-shi**
  **Chiba 274-0052 (JP)**

(74) Representative: **J A Kemp**
**14 South Square**
**Gray's Inn**
**London WC1R 5JJ (GB)**

(54) **METHOD FOR PRODUCING LIQUID MEDIUM COMPOSITION AND PRODUCTION DEVICE USED THEREFOR**

(57) A method for producing a liquid medium composition, including mixing a first liquid A1 containing a particular compound and a second liquid B1 containing a linking substance, and the method is carried out using a production apparatus. The production apparatus has a container 1 provided with at least two openings 2, 3 in a wall, the two openings are connected to each other by a tube 4 outside the container, and the tube has a part with a shape and flexibility that render the tube placeable as a pumping tube into a peristaltic pump 5. In the production method, the peristaltic pump is operated, and the first liquid and the second liquid are mixed by circulating them to form the object liquid medium composition in the container.

Fig. 1

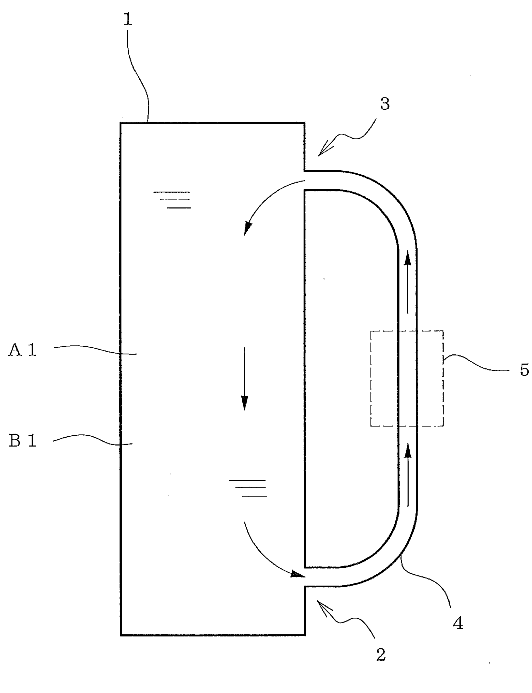


EP 3 489 341 A1

## Description

[Technical Field]

The present invention relates to a production method of a liquid medium composition and a production apparatus used for carrying out the production method. More particularly, the present invention relates to a production method including appropriately mixing at least two kinds of liquids (a first liquid containing a particular compound and a second liquid containing a substance that links the particular compound to form a structure) to be mixed for forming the above-mentioned medium composition to produce a medium composition in which the above-mentioned structures are dispersed, and a production apparatus used for preferably carrying out the production method.

[Background Art]

In recent years, techniques for proliferating or maintaining in vitro various organs, tissues and cells that play distinct roles in the body of animals and plants have been developed. Proliferation or maintenance of the organs and tissues in vitro is called organ culture and tissue culture, respectively, and proliferating, differentiating or maintaining in vitro the cells separated from an organ or tissue is called cell culture.

Cell culture is a technique for proliferating, differentiating or maintaining separated cells in vitro in a medium, and is indispensable for detailed analyses of the in vivo function and structure of various organs, tissues and cells.

In addition, the cells and/or tissues cultured by the technique are utilized in various fields including efficacy and toxicity evaluation of chemical substances, pharmaceutical products and the like, large-scale production of useful substances such as enzymes, cell proliferation factors, antibodies and the like, regenerative medicine supplementing organ, tissue and cells that were lost by disease and deficiency, improvement of plant breed, production of gene recombinant products, and the like.

As a medium for culturing cells and the like (organ, tissue, cells), a liquid culture medium can be mentioned, and the present inventors successfully developed a liquid medium composition enabling culture of cells and the like in a suspending state (patent documents 1 and 2).

The liquid medium composition described in patent document 1 is one in which particular compounds (particularly, a polymer compound having an anionic functional group) assemble via a divalent metal cation and the like to form amorphous structures, and the structures are dispersed in a liquid culture medium in a suspended state. In the following, the above-mentioned particular compound such as a polymer compound having an anionic functional group and the like is also referred to as a "particular compound ", and a substance such as a divalent metal cation and the like which binds the particular compounds is also referred to as a "linking substance".

The medium composition provides a preferable liquid culture medium capable of culturing cells and the like in a suspended state without accompanying an operation such as shaking, rotation and the like having a risk of causing damage and loss of functions of cells and the like.

[Document List]

[Patent documents]

patent document 1: WO 2014/017513
patent document 2: US-A-2014/0106348

[SUMMARY OF THE INVENTION]

[Problems to be Solved by the Invention]

The originally-intended preferable state of the liquid medium composition described in the above-mentioned patent document 1 is a state in which structures formed by coupling specific compounds with each other via a linking substance such as a divalent metal cation and the like are uniformly dispersed in the liquid culture medium.

However, the present inventors studied the actual production steps of the liquid medium composition in detail, and found that achieving such preferable state requires paying attention to the mixing method and mixing conditions to ensure that the structures are not unevenly formed locally in the medium composition.

For example, when the particular compound is deacylated gellan gum, it forms an indeterminate amorphous structure via a linking substance (e.g., calcium ion) in a liquid culture medium when mixed with the liquid medium, and

the structure becomes a carrier for suspending cells and the like.

However, in a mixing method including stirring a liquid medium containing a linking substance and pouring a liquid containing a specific compound thereinto while stirring the liquid medium may pose a problem that both liquids come into contact with the outside air and allow bacteria in the outside air to mix into the medium.

In addition, in a mixing method including placing a liquid medium containing a linking substance in a container and pouring a liquid containing a high concentration of a particular compound thereinto, the particular compound contacts the linking substance and forms a structure on the moment when the both liquids join, as a result of which the structures are sometimes linked to form a long string suspended in the mixture (or string structure entangled in a mass), which is not the originally-intended uniformly dispersed state. It was also found that such state is developed even when the mixture is stirred at a comparatively high speed. Once such string structure is formed in the liquid medium, it is not easy to cleave the structure finely and disperse same in the base material in view of the property of the structure that a double helix formed by the molecular chain forms a tight 3-dimensional network with each other via a linking substance (e.g., calcium ion).

To obtain a mixture in which the object structures are preferably dispersed, therefore, a special stirring apparatus is necessary to allow the both liquids to be contacted with each other at a high speed. However, the stirring action of such a special stirring apparatus is often generally limited to be effective for small amounts of liquids of about 3 L (L represents liter) or less, and the above structure is not suitable for producing a large amount (e.g., 20 L or more) of a mixture in which the above-mentioned structures are preferably dispersed.

An object of the present invention is to solve the above-mentioned problem and provide a production method capable of sterilely mixing any liquid containing a linking substance such as divalent metal cation and the like with a liquid containing particular compounds and producing a large amount of the liquid medium composition comprising fine structures dispersed therein, and a production apparatus used for preferably carrying out the production method.

[Means of Solving the Problems]

The main constitution of the present invention capable of achieving the aforementioned object is as follows.

[1] A method for producing a liquid medium composition, comprising mixing a first liquid containing a particular compound of the following (i) and a second liquid containing a linking substance of the following (ii), the method comprising

using a container provided with at least two openings in a wall, wherein the aforementioned at least two openings are connected to each other by a tube outside the container and wherein the tube has a part with a shape and flexibility that render the tube placeable as a pumping tube into a peristaltic pump; and

placing the aforementioned tube into the aforementioned peristaltic pump, operating the peristaltic pump, and mixing the aforementioned first liquid and the second liquid by circulating them between the aforementioned tube and the aforementioned container to form the liquid medium composition in the container, wherein the liquid medium composition comprises structures formed by the aforementioned particular compound linked via the aforementioned linking substance dispersed therein:

(i) a particular compound which is a polymer compound having an anionic functional group, and capable of forming a structure by linking via a divalent metal cation, which structure being capable of suspending a cell or a tissue,

(ii) a linking substance which is a divalent metal cation.

[2] The production method of the aforementioned [1], wherein

the particular compound of (i) is deacylated gellan gum, the first liquid is an aqueous solution containing deacylated gellan gum,

the linking substance of (ii) is a concentrated liquid comprising one or both of calcium ion and magnesium ion, and the second liquid is a liquid culture medium containing one or both of calcium ion and magnesium ion.

[3] The production method of the aforementioned [2], wherein a concentration of the deacylated gellan gum in the liquid medium composition is 0.001% (w/v) to 1.0% (w/v).

[4] The production method of any of the aforementioned [1] to [3], wherein a volume mixing ratio of the first liquid and the second liquid is 50 - 200 volume of the second liquid relative to 100 volume of the first liquid.

[5] The production method of any of the aforementioned [1] to [4], wherein the aforementioned one of the openings is provided as an outlet in a wall constituting a bottom surface of the aforementioned container or a bottom of a wall

constituting a side surface thereof,

the aforementioned other opening is provided as an outlet in a wall constituting a top surface of the aforementioned container or an upper part of the wall constituting the side surface,
the aforementioned peristaltic pump is operated, the first liquid and the second liquid move in the tube from the aforementioned outlet toward the aforementioned inlet, by which the first liquid and the second liquid in the container are moved in a direction from the top to the bottom.

[6] The production method of any of the aforementioned [1] to [5], wherein said two openings are paired as an outlet and an inlet, said container is provided with a plurality of pairs of said two openings, and the two openings in each pair are connected to each other by a tube, and
in the aforementioned second step, each tube is placed into a separate peristaltic pump, the peristaltic pump is operated, and the aforementioned first liquid and the second liquid are circulated through each tube.
[7] The production method of any of the aforementioned [1] to [6], wherein the aforementioned container is made of a flexible material so that the volume of the container may vary according to the volume of the liquid contained inside the container, whereby the liquid is injected into the container and discharged to the outside of the container while securing the airtightness in the container.
[8] The production method of any of the aforementioned [1] to [7], wherein the aforementioned container is provided with an opening for an entrance used for putting the liquid into and out from the container,
one of the first liquid and the second liquid is placed in advance in the aforementioned container, and the other liquid is injected into the container from the aforementioned opening for an entrance while circulating the liquid by the aforementioned peristaltic pump.
[9] A production apparatus used for practicing the production method of the aforementioned [1], comprising

a container for containing the first liquid and the second liquid to be mixed in the aforementioned production method, wherein
at least two openings are provided in a wall of the container, and these openings are connected to each other by a tube outside the container, and
the aforementioned tube has a part with a shape and flexibility that render the tube placeable into a peristaltic pump configured to deliver a liquid in the tube and to be operable as a pumping tube in the peristaltic pump.

[10] The production apparatus of the aforementioned [9], wherein the aforementioned one of the openings is provided in a wall constituting a bottom surface of the aforementioned container or a bottom of a wall constituting a side surface, and
the aforementioned other opening is provided in a wall constituting a top surface of the aforementioned container or an upper part of a wall constituting a side surface.
[11] The production apparatus of the aforementioned [9] or [10], wherein said two openings are paired as an outlet and an inlet, said container is provided with a plurality of pairs of said two openings, and the two openings in each pair are connected to each other by a tube.
[12] The production apparatus of any of the aforementioned [9] to [11], further comprising the aforementioned peristaltic pump as a constituent element of the production apparatus, wherein the aforementioned tube is placed in the peristaltic pump and operated as a pumping tube.
[13] The production apparatus of the aforementioned [12], wherein the aforementioned one of the openings is provided in a wall constituting a bottom surface of the aforementioned container or a bottom of a wall constituting a side surface,

the aforementioned other opening is provided in a wall constituting a top surface of the aforementioned container or an upper part of a wall constituting a side surface, and
the aforementioned peristaltic pump is configured to operate such that a liquid in the aforementioned tube moves from an opening on a lower side to an opening on an upper side, whereby the liquid contained in the container moves downward from above.

[14] The production apparatus of any of the aforementioned [9] to [13], wherein the aforementioned container is made of a flexible material so that the volume of the container may vary according to the volume of the liquid contained inside the container, whereby the liquid is injected into the container and discharged to the outside of the container while securing the airtightness in the container.
[15] The production apparatus of any of the aforementioned [9] to [14], wherein the aforementioned container is further provided with an opening for an entrance used for putting the liquid into and out from the container.

[Effect of the Invention]

According to the production method of the present invention (hereinafter to be also referred to as the production method), by the action of a peristaltic pump 5 on a tube 4 in Fig. 1, the first liquid and the second liquid can be circulated through a closed pathway of [exit from the container into the tube, pass through the tube and return to the original container]. The circulation in the closed pathway makes it possible to mix the both liquids without allowing them to contact a contamination source such as outside air and the like or contact with a member other than the container and the tube (including connector parts for connecting these).

Hereinafter, the movement of the liquid in the container to exit from one opening into the tube by the operation of the peristaltic pump, pass through the tube, and circulate back into the container from the other opening is also referred to as "the liquid circulates between the container and the tube", and simply as "the liquid circulates".

In addition, according to the production method, a large amount of liquid medium composition in which the total of both liquids reaches 100 L can be produced aseptically and efficiently.

In addition, the production apparatus of the present invention (hereinafter to be also referred to as the production apparatus) having a configuration including a container and a tube enables the production method to be carried out, and allows the aforementioned aseptic and large-volume mixing to be carried out. Especially, when the container is a bag made of an organic polymer material such as a flexible synthetic resin, and the like, the entire apparatus can be composed of an inexpensive bag and a tube, and can be disposed for each mixing. This eliminates the need for cleaning of the inside of the container and the inside of the tube, and further increases the sterility of the inside of the container and the inside of the tube.

In addition, even if the production apparatus essentially includes a peristaltic pump, the tube is merely placed into the driving part of the peristaltic pump, and is not connected integrally. Therefore, the main part of the circulation path including the container and the tube can be disposed for each mixing, and cleaning of the inside of the container and the inside of the tube is not necessary.

[Brief Description of the Drawings]

Fig. 1 is a schematic showing of the production method and production apparatus of the present invention.
Fig. 2 shows a configuration example of the production apparatus of the present invention.
Fig. 3 shows another configuration example of the production apparatus of the present invention.
Fig. 4 shows a still other configuration example of the production apparatus of the present invention. Fig. 4(a) shows one of the end surfaces of the container. Fig. 4(b) shows the other end surface of the container. Fig. 4(c) is a block diagram showing a connection structure of the entire production apparatus, in which the container is shown in a perspective view. In Fig. 4(c), a hidden portion is shown with a dashed line, and a dashed line is also used as a lead line to impart a symbol to the hidden part.
Fig. 5 shows one embodiment of the structure of a peristaltic pump used for the production method and production apparatus of the present invention.
Fig. 6 is a schematic showing of the criteria for evaluating the mixing state of the liquid medium composition produced in the Examples of the present invention.

[Description of Embodiments]

Hereinafter, the production method of the present invention is described in detail, reference is made to the production apparatus of the present invention, and the structure of the production apparatus is also described in detail.

The production method of the present invention is a method for producing the liquid medium composition by mixing the first liquid containing a particular compound of the following (i) and the second liquid containing a linking substance of the following (ii). Details of the particular compound, linking substance, and the first liquid and the second liquid respectively containing them are described later.

(i) a particular compound which is a polymer compound having an anionic functional group, and capable of forming a structure by linking via a divalent metal cation, which structure being capable of suspending a cell or a tissue,
(ii) a linking substance which is a divalent metal cation.

In the production method, a container 1 is used as exemplified in Fig. 1, which has at least two openings 2, 3 provided in a wall. The aforementioned at least two openings 2, 3 are connected to each other by a tube 4 outside the container. The tube 4 contains, as part or all of the tube, a part having a shape and flexibility that render the tube to be

placed as a pumping tube into a peristaltic pump 5.

In Fig. 1, a liquid in the container 1 is not depicted to be clearly separated into the first liquid A1 and the second liquid B1. It is suggested that the first liquid A1 and the second liquid B1 are being mixed by assigning the symbols A1, B1 to the liquid in the container.

At least two openings 2, 3 are provided in the wall of the aforementioned container 1, and these at least two openings 2, 3 are connected to each other by a tube 4 outside the container 1. The tube 4 has a shape and flexibility that render the tube placeable into the peristaltic pump 5 and functionable as a pumping tube. In Fig. 1, a detailed structure of the peristaltic pump 5 is not shown, and the presence of the peristaltic pump is suggested with a dashed line. The peristaltic pump 5 may be contained as a part of the production apparatus of the present invention or may be an outside apparatus.

In the production method, as shown in Fig. 1, the tube 4 is placed into the peristaltic pump 5. That is, the tube 4 is engaged with the peristaltic pump 5 such that the tube 4 is the pumping tube of the peristaltic pump 5. Then, the peristaltic pump 4 is operated to circulate the first liquid A1 and the second liquid B1 between the container 1 and the tube 4. The circulation stirs and mixes the both liquids to form the liquid medium composition. In the liquid medium composition in which the both liquids have been sufficiently stirred and mixed, a structure in which a particular compound is bound via a linking substance is uniformly dispersed, and the composition is suitable for culturing cells and the like in a floating state.

The production method makes it possible to handle any large quantities of the first liquid A1 and the second liquid B1 such as a sum of the both liquids reaching 1000 L, as described above, and to sufficiently mix the both liquids by a simple operation without allowing contact with a contamination source such as the outside air.

The production apparatus of the present invention is an apparatus used for performing the production method of the aforementioned present invention. The production apparatus contains, as shown in Fig. 1, a container 1 for containing the first liquid A1 and the second liquid B1 to be mixed in the production method. At least two openings 2, 3 are provided in the wall of the container 1, and these at least two openings are connected to each other by a tube 4 outside the container. As a result, a circulation flow path is formed in which a liquid contained in the container 1 can exit from one of the openings 2, passes through the tube 4 and returns from the other opening 3 to the aforementioned container 1. The tube 4 has properties as a pumping tube that render the tube placeable into the peristaltic pump 5 configured to deliver the liquid in the tube. By placing the tube into the peristaltic pump and operating the peristaltic pump, the liquid in the container is circulated between the container 1 and the tube 4 without contacting a contamination source such as outside air and the like and is thoroughly mixed to give the desired liquid medium composition.

The above-mentioned container 1 which can be used in the production method of the present invention and the production apparatus of the present invention is preferably made of a material which can be accommodated without an influence on the first liquid and the second liquid.

The volume of the container is not particularly limited; however, a large volume clarifies the significant advantage of the present invention that large quantities of the both liquids can be mixed. A preferable volume of the container is not particularly limited and may be appropriately determined according to requirements. For example, about 100 L to 1000 L is recited as an example of a preferable volume for mass production of a liquid medium for general cell culture.

The material of the above-mentioned container 1 is not particularly limited, and may be, for example, a hard or soft organic polymer material (particularly a synthetic resin material etc.) such as glass, metal or the like. The hard or soft organic polymeric material may be a plate or a film. The above-mentioned plate or film may be made of a single material (single layer) or may be a multilayer structure (two or more layers) including layers made of different materials, as described later.

Also, the shape of the container 1 is not particularly limited, and may be, for example, a cube, a rectangular parallelepiped, a cylinder, a bag having a wall surface made of a film made of a soft organic polymer material (for example, a bag having outer peripheral edges of two films bonded to each other, a bag having a bottom surface and a side surface, a bag having a top surface and a bottom surface and a side surface and the like), or the like.

When the container 1 is a flexible bag made of a film or the like made of a soft organic polymer material, the following preferable effects (a) to (c) can be obtained.

(a) The container can be shrunk to evacuate the inside air before injecting the first liquid or the second liquid into the container 1 for the first time. Since the container 1 can expand as the liquid is injected, the injected liquid does not come into contact with air.

(b) A mixture (liquid medium composition) thoroughly mixed by circulating the first liquid and the second liquid can be removed from the container 1 into an external container while avoiding contact with air by contracting the container.

(c) When the container 1 is a soft bag made of a soft organic polymer material film or the like, the container can be obtained at a low cost. Thus, the container and the tube can be thrown away after every mixing, the washing time in the production apparatus can be saved, as well as the sterility inside the container and the tube becomes higher.

The aforementioned flexible bag made of the film made of a soft organic polymer material includes those similar to a bag for cell culture known in the art. Examples of the material of the film constituting the wall of such flexible bag for cell culture include an ethylene-vinyl acetate copolymer resin (EVA resin), a polyethylene-based resin, a polypropylene-based resin, a polyurethane-based resin, a polyester-based resin, a polyamide-based resin, a vinyl chloride-based resin and the like. When the film has a multilayer structure, the material of each layer may be appropriately selected according to the function. For example, in the case of a three-layer structure, a combination of (polyethylene terephthalate layer (inner layer)/polyethylene layer (intermediate layer)/nylon layer (outer layer)) is exemplified.

The thickness of the wall is not particularly limited, and is generally about 50 $\mu$m - 150 $\mu$m, preferably about 100 $\mu$m.

As the overall form of the aforementioned flexible bag-like article, a bag-like article in which the outer peripheral edges of two films are joined to each other, a bag-like article having a bottom surface and a side surface, a bag-like article having a top surface, a bottom surface and a side surface, and a bag-like article having various shapes (a rectangular parallelepiped, a hexagonal prism (shown in Fig. 4), a polygonal prism, a cylinder, and the like) can be recited as examples.

The aforementioned flexible bag may be a commercially available bag for cell culture, or may be manufactured using the aforementioned film as a container dedicated to the present invention. In either case, the production apparatus according to the present invention is obtained by forming at least two openings in the wall of the container, connecting the openings by a tube, and forming a closed circulation path.

As exemplified in Fig. 1, at least two openings 2, 3 are provided in the wall of the container 1. One of the openings of the at least two openings (lower opening 2 in the Figure) is the outlet through which the liquids A1, B1 exit from the container 1 into the tube (circulation tube) 4, and the other opening (upper opening 3 in the Figure) is the inlet through which the liquid enters into the container 1 from the circulation tube 4.

One or both of these at least two openings circulation tube may be used, after removing the circulation tube, as an opening for injecting the first liquid and the second liquid into the container or may be used as an opening for taking out a mixture of the both liquids from the inside of the container.

The aforementioned two openings 2 and 3 used for circulation of the liquid are preferably connected aseptically by the circulation tube 4 so that they will not be open to the outside. On the other hand, the tube (injection tube) for injecting the liquid into the container is in contact with the liquid before filtration sterilization. Thus, if it is cut off at the tube part after filter sterilization and used by connecting for circulation, the content liquid may be contaminated.

From the above points, it is preferable to provide the necessary number of openings for injecting the first liquid and/or the second liquid into the container as separate openings from the two openings for circulation. For example, as shown in Fig. 3, only one opening 6 as an exclusive entrance may be provided and the first liquid and the second liquid may be sequentially injected into the container from the opening 6. Alternatively, two or more openings may be provided as shown in Fig. 4, and the first liquid and the second liquid may be simultaneously injected into the container. The "opening as an entrance" here means an opening for an entrance for injecting a liquid from the outside into the container, and also means an opening for an outlet for discharging the mixture (liquid medium composition) after mixing to the outside of the container. For example, the opening for the entrance is provided, the first liquid and the second liquid are injected into the container through the opening for the entrance, and the mixture after mixing may be taken out, for circulation, from the opening for circulation. Also, for example, the opening for the outlet may be provided and the first liquid and the second liquid may be injected from the opening for circulation into the container and the mixture after mixing may be taken out from the opening exclusively for the outlet. In addition, the opening for the inlet and the opening for the outlet may be separately provided on the container.

To maintain sterility in the production apparatus, it is preferable to insert a filtration filter in the middle of the injection tube and inject a filtered liquid into the container. For example, it is possible to adopt a configuration in which a cartridge type filter is incorporated in the tube 4c shown in Fig. 3 and inserted into the route.

In addition, since the container, tube, sterilization filter, and connector may be contaminated by the outside air when connecting them, it is preferable to perform gamma irradiation sterilization after all of the container, tube, sterilization filter, and connector are joined to form a closed production apparatus.

Fig. 2 shows another example of the position of the opening provided in the container. In this Figure, connector and coupling for connecting the container and the tube are not shown. In the present invention, the tube 4 is connected to the openings 2 and 3 provided in the wall of the container, the first liquid and the second liquid are circulated by the action of the peristaltic pump, and the both liquids are mixed. Thus, the both liquids are more efficiently mixed when the opening 2 serving as the outlet and the opening 3 serving as the inlet are separated as far as possible from each other. When the outlet and the inlet are close to each other, only the liquid in the vicinity of these openings locally circulates, and the remaining liquid in the container may be highly possibly left behind without being added to the circulation flow.

In the embodiment of Fig. 2(a), the opening 2 serving as the outlet is provided in a wall constituting the lower surface of the container, the opening 3 serving as the inlet is provided in the wall constituting the upper surface, and these are connected by the tube 4. As a result, a flow from the upper side to the lower side is generated in the container

as indicated by an arrow, and the first liquid and the second liquid are efficiently mixed.

In the embodiment of Fig. 2(b), the opening 2 serving as the outlet is provided in one wall of the container, the other opening 3 is provided in the wall opposed to the aforementioned wall, and these are connected by the tube 4. As a result, a horizontal flow is generated in the container as indicated by an arrow in the container, and the first liquid and the second liquid are mixed with no portion left unmixed.

In the embodiment of Fig. 2(c), a plurality of openings (two of 2a, 2b in the example in the Figure) serving as outlets are provided in one wall (wall constituting the bottom surface of the container in the example of the Figure) of the container, a plurality of openings (two of 3a, 3b in the example in the Figure) serving as inlets are provided in a wall opposed to the aforementioned wall (wall constituting the upper surface of the container in the example of the Figure), these are multiple pairs of inlets and outlets, each pair of openings (2a, 3a), (2b, 3b) are respectively connected by tubes 4a, 4b, and the tubes 4a, 4b are respectively mounted on peristaltic pumps 5a, 5b. As a result, a wide parallel flow from top to bottom occurs in the container as indicated by an arrow in the container, the liquid in the container is highly possibly added to the circulation flow, and the first liquid and the second liquid are efficiently mixed. As in the embodiment of Fig. 2(c), when plural tubes are connected, intentional turbulence may be produced in the flow in the container with the direction in which the liquid moves in one tube (e.g., rising direction in Fig. 2(c)) and the direction in which the liquid moves in the other tube as a reverse direction (e.g., downward direction in Fig. 2(c)).

The direction of the current that leads from one opening to the other opening when the peristaltic pump is operated is, as shown in Fig.1, Fig. 2 (a) to (c), a vertical (downward, upward) direction, horizontal direction, diagonal direction, or other direction that causes a turbulent flow. When the first liquid is an aqueous solution containing deacylated gellan gum, there is a tendency for deacylated gellan gum to rise up in the culture medium. A flow in which a liquid descends from the top to the bottom of the container, as shown in Fig. 1, Fig. 2(a), Fig. 2(c), Fig. 4, is a favorable flow that makes mixing proceed more effectively against such rising up.

The arrangement and the number of the openings shown in Fig. 1, Fig. 2(a) - (c) are mere examples, and these may be changed in direction and combined appropriately. For example, in the embodiment of Fig. 1, two openings 2, 3 are provided in a vertical wall of the container. However, these two openings may be provided on the wall of the upper surface (or the bottom surface) of the container, or the posture of the container of Fig. 1 may be changed and used. In the embodiment of Fig. 1, two openings 2, 3 are provided only in a wall on the right side of the container. However, a pair of openings may also be provided in the wall on the left side which is opposed to the wall, and each pair of openings may be connected to each with the tube. Furthermore, an opening provided in the upper surface of the container and an opening provided in the side surface of the container may be connected as a pair by the tube.

The embodiment of Fig. 2(a) - (c) is also the same, and the attitude of the container may be appropriately changed and used.

The opening provided in the container does not necessarily have a pair of two openings as the outlet and the inlet, and one opening (e.g., an outlet) and a plurality of openings (e.g., an inlet) may correspond to each other by branching the tube (not shown).

While the diameter of the opening is not particularly limited, it is preferably equal to the diameter of the tube to be connected. Here, the diameter of the opening is an inner diameter of a flow path of the connector or coupling when the connector or coupling for connecting the tube is mounted to the opening. To maintain sterility within the container, all openings are preferably closed until use by closures, on-off valves, connectors, couplings, sealing members, and the like. It is preferable that the connector or coupling has a structure in which the flow path is closed until each mating connector or coupling is connected, and has a structure in which the flow path is aseptically communicated by connecting the mating connector or coupling.

Instead of the aforementioned connector or coupling, a short tube may be coupled to the opening, and the outside exit of the tube may be closed by the aforementioned connector or coupling.

The tube can be placed as a pumping tube into a peristaltic pump to be described later and has a part having a shape and flexibility enabling function and operation as a pumping tube.

Being able to be placed as the pumping tube into the peristaltic pump and having the shape and flexibility enabling function and operation as the pumping tube at least means that the pump has an inner diameter, outer shape, and length of the tube that are compatible with the peristaltic pump used, and has a softness that allows crushing when pressed against an actuator (roller etc.) of the peristaltic pump, and an elasticity that can restore the original shape when released from pressure by the actuator.

The entire tube may be the pumping tube, or only the portion attached to the peristaltic pump may be the pumping tube.

By placing the part to be a pumping tube into a peristaltic pump and operating the peristaltic pump, the aforementioned first liquid and the second liquid move in the tube. As a result, the both liquids are circulated and mixed, and the liquid medium composition containing structures formed by the particular compound linked via the linking substance uniformly dispersed therein is formed in the container.

The embodiment of the tube is not particularly limited except for the portion to be a pumping tube, and may be

a metal tube or the like. It is preferable that the tube is a flexible and inexpensive resin tube which does not affect the first liquid or the second liquid and can be used for cell culture. An embodiment in which the entire tube can be used as a pumping tube is one preferred example.

The material of the portion to be the pumping tube is preferably a resin material that does not affect the first liquid and the second liquid, is usable for cell culture, and has elasticity and flexibility, particularly a synthetic resin material. Examples thereof include a styrene-based thermoplastic elastomer, a silicon-based resin, a fluorine-based resin, a polyvinyl chloride-based resin, and the like.

The inner diameter, thickness and length of the part to be the pumping tube may be any as long as they correspond to those of the peristaltic pump to be used, and the inner diameter is about 5 mm - 50 mm and the thickness of the tube is about 2 mm - 6 mm. In one embodiment, the tube used in the Examples had an inner diameter of 13 mm and a thickness of 3 mm (outside diameter 19 mm).

The entire length of the tube may be appropriately determined according to the distance between the openings to be connected and the size of the container, and it is, for example, about 1500 mm - 3000 mm.

The circulation tube may be a single tube which integrally continues from a portion connected to one opening of the container to a portion connected to the other opening, or may be a single tube which is appropriately connected by a connector or a coupling. Connectors and couplings may be provided at both ends of the portion to be a pumping tube, and may be detachable from the entire tube, in order to improve the handling property when connecting to the peristaltic pump. As such connectors and couplings, connectors that can be connected aseptically and are referred to as sterile connectors or the like are preferably used.

Fig. 3 shows another configuration example of the production apparatus. In the example of this Figure, similar to the example of Fig. 2(c), two openings (2a, 2b) serving as outlets are provided in the wall of the bottom surface of the container 1, and two openings (3a, 3b) serving as inlets are provided in a wall of the top surface of the container, and these are two pairs of inlets and outlets, each pair of openings (2a, 3a), (2b, 3b) are respectively connected by tubes 4a, 4b, and the tubes 4a, 4b are respectively mounted on peristaltic pumps 5a, 5b. An opening 6 for an entrance is further provided on the wall surface (upper surface in the example of the Figure) of the container 1 such that a liquid can be injected into the container 1 from an external container 7 through tube 4c.

With the configuration exemplified in Fig. 3, it is possible to perform a procedure of circulating one liquid with a peristaltic pump and injecting the other liquid thereinto. In particular, as shown in Fig. 3, it is possible to perform a mixed procedure of first storing the second liquid B1 and injecting thereinto the first liquid A1 from the external container 7 at a predetermined flow rate while circulating the second liquid B1.

In moving a liquid from the external container 7 to the container 1, tube 4c may be attached to the peristaltic pump, and the feeding ability of the peristaltic pump may be utilized. Alternatively, the external container 7 may be disposed above the container 1 and the liquid in the external container 7 may be moved inside the tube by gravity and dropped into the container 1.

Fig. 4 shows a preferable configuration example of the production apparatus.

The overall shape of the container 1 is a flexible bag having a hexagonal prism as shown in Fig. 4(c). In the example shown in the Figure, the flexible bag itself exhibiting the hexagonal prism is a commercially available cell culture bag. Fig. 4(a) shows one end face 1a of the container 1 and Fig. 4(b) shows the other end face 1b of the container 1. The both end faces 1a and 1b have a hexagonal shape as the end face of the hexagonal prism, and the attitude of the container is maintained such that the both end faces are vertical planes.

As shown in Fig. 4(a), an opening 6a for allowing the first liquid to flow into the container 1 and an opening 6b for allowing the second liquid to flow into the container 1 are provided at the upper part of the end face 1a. The opening 6c is closed and it can be used for taking out the mixture and the like. On the other hand, as shown in Fig. 4(b), openings 2 and 3 for circulation are respectively provided at the lower part and the upper part of the end face 1b. In the example of the Figure, a connector for connecting a tube serving as a conduit is provided in all the openings.

Fig. 4(c) is a block diagram showing the connection configuration of the production apparatus as a whole. In the end face 1a of the container 1, the external container 7a containing the first liquid is connected to the opening 6a via a conduit (tube) 41, and the external container 7b containing the second liquid is connected to the opening 6b via a tube 42. These outside containers 7a, 7b may be included as an element of the production apparatus or may be regarded as an external liquid supply source.

The tube connected to the opening 6a and a tube connected to the external container 7a are connected to one tube 41 by an aseptic connector C1. Similarly, the tube connected to the opening 6b and the tube connected to the external container 7b are connected to one tube 42 by an aseptic connector C2. On the other hand, on the end face 1b of the container 1, a tube 43 is connected to the opening 2, the tube 43 is connected to the pumping tube 44 by a sterile connector C3, the pumping tube 44 is attached to and passes through the roller pump 5 and is connected to the tube 45 by a sterile connector C4, and the tube 45 is connected to the opening 3.

In the example of Fig. 4(c), the second liquid contained in the external container 7b is injected into the container 1 in a state of shrinkage after deflation. The container 1 expands by the amount of the injected second liquid. Subsequently,

the roller pump 5 is operated, the first liquid contained in the external container 7a is injected into the container 1 while the second liquid is circulated, and mixing proceeds by the circulation thereafter. The roller pump 5 moves the compression point on the pumping tube 44 so that the liquid will move from the opening 2 toward the opening 3. As a result, the liquid in the container 1 moves from the upper side to the lower side.

The peristaltic pump which can be used in the present invention is a pump having a function of moving a liquid in a pipe line by moving the position, at which a pumping tube having elasticity and flexibility is crushed, in a feeding direction, as typified by a tube pump (also called a roller pump).

Fig. 5 schematically shows one configuration embodiment of the peristaltic pump. In the example of this Figure, an arcuate or semicircular outer peripheral wall surface 11a is provided in the housing 11, and the pumping tube 4 is placed in a curved manner along the outer peripheral wall surface 11a. The outer peripheral wall surface 11a supports the pumping tube 4 on the outer peripheral side. On the inner circumference side of the curvature of the pumping tube 4, the roller 13 supported by the arm 12 rotates about the rotation axis 14 and in the liquid feed direction (direction of arrow f). As a result, as in a peristaltic motion, the pumping tube 4 is sequentially compressed in the liquid feed direction and the liquid in the pumping tube is delivered. Placing a tube into a peristaltic pump means, as exemplified in Fig. 5, that the tube 4 is set by insertion through a feed drive portion (portion that successively compresses the tube) of the peristaltic pump so that the tube 4 will function as a pumping tube of the peristaltic pump.

In the example of Fig. 5, while two rollers 13 are provided at both ends of the linear arm 12, a more number of rollers may be provided depending on the peristaltic pump, or a single large roller without an arm may rotate around an eccentric axis. The peristaltic pump may have a structure in which the path is curved as shown in Fig. 5, or a structure in which a number of linearly arranged actuators locally and sequentially compress the pumping tube to apply a peristaltic motion to the pumping tube. For details of the feed structure of the peristaltic pump, reference can be made to the prior art.

The feed capability of the peristaltic pump (flow rate per minute of the liquid moving in the tube) widely varies from a very small flow rate to a large flow rate in commercially available pumps. Examples include about 0.01(mL/min) - 10(L/min). The feeding capacity of the peristaltic pump utilized in the present invention is not particularly limited and may be determined according to the volume of the container. When the volume of the container is about 20 L - 100 L, the feed capability of the peristaltic pump is exemplified by a preferable range of about 3 - 18 (L/min).

To increase the flow rate of the circulation of the liquids for mixing the first and second liquids, a pumping tube with a larger inner diameter and a peristaltic pump with a larger feeding capacity may be used, or a pair of tubes with a smaller feeding capacity and a peristaltic pump may be used in parallel in the required number of pairs to circulate the liquids in parallel.

When the flow rate of the liquid by the peristaltic pump is excessively small relative to the volume of the container, mixing may take a long time, and a state of insufficient mixing may occur locally.

The operation time of the peristaltic pump (that is, time of mixing by continuous circulation) can be determined as appropriate according to the feed amount of the peristaltic pump and based on the mixing state. As one example, when the volume of the container is about 50 L to 150 L and the feed capability of the peristaltic pump is about 10 (L/min), a preferable operation time is exemplified by about 2 - 4 hr.

The peristaltic pump may form a part of the production apparatus of the present invention or may be an external device to be utilized by the apparatus of the present invention.

When the first liquid and the second liquid are circulated and mixed by the peristaltic pump, one of the first liquid and the second liquid is first placed in the container, and the other liquid is injected into the container while the one liquid is circulated by the peristaltic pump, whereby the both are preferably mixed by the subsequent circulation.

When the first liquid is an aqueous solution containing the deacylated gellan gum and the second liquid is a liquid containing a linking substance which is a divalent metal cation (e.g., liquid medium containing calcium ion and/or magnesium ion), a procedure in which one liquid is circulated by the peristaltic pump and the other liquid is injected into the container is preferable. This affords an effect that the deacylated gellan gum is less susceptible to local distribution in the culture solution, compared to when circulation is started after injecting in advance the both liquids into the container at a predetermined ratio. Particularly, the deacylated gellan gum tends to be more preferably dispersed when the second liquid (the aforementioned liquid medium) is previously stored in a container and the first liquid (aqueous solution containing the deacylated gellan gum) is injected into the container while circulating the second liquid by the peristaltic pump.

In one embodiment of mixing, when the volume of the container is about 50 L - 150 L, the volume ratio of the first liquid and the second liquid is 1:1, and the feed capability of the peristaltic pump is about 10 (L/min), it is preferable to store one of the liquids in the container 1, activate the peristaltic pump, inject the whole amount of the other liquid into the container 1 in about 0.5 hr - 1.0 hr while circulating the earlier liquid, and further continue circulation of the mixture.

In the case of a procedure of circulating one liquid with the peristaltic pump while injecting the other liquid into the container, it is preferable to additionally provide an opening 6 for injecting the liquid into the container later, in addition to the two openings for circulating the liquid, as shown in the configuration example of the production apparatus in Fig. 3. The opening 6 may be for the inlet.

The peristaltic pump may be controlled to operate and stop manually, or the production apparatus may be

provided with a control equipment, and the control device may control the peristaltic pump to operate automatically for a predetermined time.

[the first liquid]

The first liquid contains, as a particular compound, a polymer compound having an anionic functional group and capable of forming a structure that can suspend cells or tissues by binding via a divalent metal cation.

As the anionic functional group, a carboxy group, sulfo group, phosphate group and a salt thereof can be mentioned, with preference given to carboxy group or a salt thereof. A polymer compound to be used in the present invention may contain one or more kinds selected from the group of the aforementioned anionic functional groups.

Specific preferable examples of the polymer compound to be used in the present invention include, but are not limited to, polysaccharides wherein not less than 10 monosaccharides (e.g., triose, tetrose, pentose, hexsauce, heptose etc.) are polymerized, more preferably, acidic polysaccharides having an anionic functional group. The acidic polysaccharides here is not particularly limited as long as it has an anionic functional group in the structure thereof, and includes, for example, polysaccharides having a uronic acid (e.g., glucuronic acid, iduronic acid, galacturonic acid, mannuronic acid), polysaccharides having a sulfate group or phosphate group in a part of the structure thereof, and polysaccharides having the both structures, and includes not only naturally-obtained polysaccharides but also polysaccharides produced by microorganisms, polysaccharides produced by genetic engineering, and polysaccharides artificially synthesized using an enzyme. More specifically, examples thereof include polymer compounds composed of one or two or more kinds from the group consisting of hyaluronic acid, gellan gum, deacylated gellan gum (hereinafter sometimes to be referred to as DAG), rhamsan gum, diutan gum, xanthan gum, carageenan, xanthan gum, hexuronic acid, fucoidan, pectin, pectic acid, pectinic acid, heparan sulfate, heparin, heparitin sulfate, keratosulfate, chondroitin sulfate, dermatan sulfate, rhamnan sulfate and a salt thereof. Polysaccharides are preferably hyaluronic acid, DAG, diutan gum, xanthan gum, carageenan or a salt thereof, more preferably DAG or a salt thereof. Phosphorylated DAG can also be used. The phosphorylation can be performed by a known method.

The salt here includes, for example, salts with alkali metal such as lithium, sodium, potassium; salts with alkaline earth metals such as calcium, barium, magnesium; and salts with aluminum, zinc, copper, iron, ammonium, organic base and amino acid and the like.

The weight average molecular weight of these polymer compounds (polysaccharides etc.) is preferably 10,000 to 50,000,000, more preferably 100,000 to 20,000,000, still more preferably 1,000,000 to 10,000,000. For example, the molecular weight can be measured based on pullulan by gel penetration chromatography (GPC).

In the present invention, plural kinds (preferably two kinds) of the above-mentioned polysaccharides having an anionic functional group can be used in combination. A polysaccharide having an anionic functional group and a polysaccharide not having an anionic functional group may also be combined. The kind of the combination of the polysaccharides is not particularly limited as long as the aforementioned structure is formed in a liquid culture medium by linking via a divalent metal cation. Preferably, the combination includes at least DAG or a salt thereof. That is, a preferable combination of polysaccharides contains DAG or a salt thereof, and polysaccharides other than DAG and a salt thereof (e.g., xanthan gum, alginic acid, carageenan, diutan gum, methylcellulose, locust bean gum or a salt thereof). Examples of specific combination of polysaccharides include, but are not limited to, DAG and rhamsan gum, DAG and diutan gum, DAG and xanthan gum, DAG and carageenan, DAG and xanthan gum, DAG and locust bean gum, DAG and κ-carageenan, DAG and sodium alginate, DAG and methylcellulose and the like.

The deacylated gellan gum is a linear high molecular weight polysaccharide containing 4 molecules of sugars of 1-3 bonded glucose, 1-4 bonded glucuronic acid, 1-4 bonded glucose and 1-4 bonded rhamnose as the constituent unit, which is a polysaccharide of the following formula (I) wherein $R_1$, $R_2$ are each a hydrogen atom, and n is an integer of two or more. $R_1$ may contain a glyceryl group, $R_2$ may contain an acetyl group, and the content of the acetyl group and glyceryl group is preferably not more than 10%, more preferably not more than 1%.

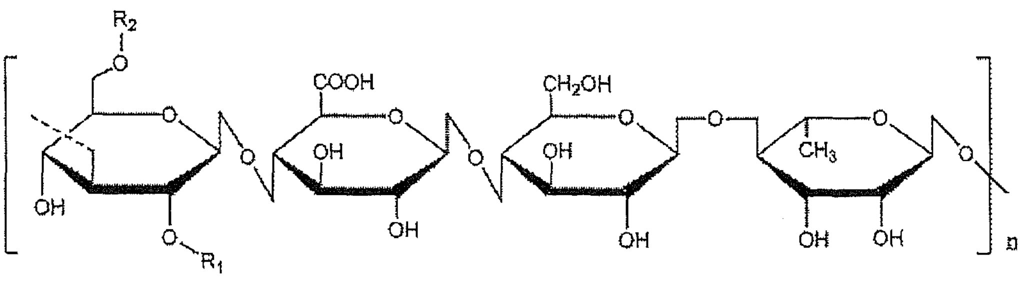

(I)

The particular compound may be obtained by a chemical synthesis method. When the particular compound is

a naturally-occurring substance, it may be obtained from various plants, various animals, various microorganisms containing the compound by extraction, separation and purification by conventional techniques. For example, gellan gum can be produced by culturing producing microorganisms in a fermentation medium, recovering mucous products produced outside the bacterial cells by a general purification method, and, after the processes of drying, pulverizing and the like, powderizing the products. In the case of deacylated gellan gum, an alkali treatment should be applied when the mucous products are recovered, to deacylate the glyceryl group and the acetyl group bonded to 1-3 bonded glucose residue, and then the given products are recovered. Examples of the gellan gum-producing microorganism include, but are not limited to, Sphingomonas elodea and microorganism obtained by altering the gene of Sphingomonas elodea.

In the case of deacylated gellan gum, commercially available products, for example, "KELCAOGEL (registered trade mark of CP Kelco) CG-LA" manufactured by SANSHO Co., Ltd., "KELCOGEL (registered trade mark of CP Kelco)" manufactured by San-Ei Gen F.F.I., Inc. and the like can be used. As a native type gellan gum, "KELCOGEL (registered trade mark of CP Kelco) HT" manufactured by San-Ei Gen F.F.I., Inc. and the like can be used.

The first liquid is generally a solution of the particular compound. While the solvent for the solution is not particularly limited as long as it can dissolve the particular compound, it is generally water or hydrophilic solvent, preferably water. In a preferable embodiment, therefore, the first liquid is an aqueous solution of the particular compound.

The concentration of the particular compound contained in the first liquid is not particularly limited as long as, upon mixing with the second liquid, the particular compounds are linked via a divalent metal cation to form a structure capable of suspending cells or tissues in the mixture, the structure is uniformly dispersed in the mixture, and further, the finally-obtained liquid medium composition containing the structure can culture the cells or tissue in suspension. The concentration of the particular compound in the first liquid can be calculated from the concentration of the particular compound in the medium composition capable of culturing cells or tissues in suspension and the ratio of the volume of the first liquid to the volume of the medium composition obtained as the final product, as described in detail below. For example, when the first liquid with volume $V_1$ and the second liquid with volume $V_2$ are mixed to finally obtain the liquid medium composition with volume $V_1+V_2$, the concentration C%(w/v) of the particular compound in the liquid medium composition can be achieved by setting the concentration of the particular compound in the first liquid to $C\times (V_1+V_2)/V_1$% (w/v).

The concentration of the divalent metal cation in the first liquid needs to be lower than the concentration at which the particular compound forms the structure in the first liquid. Examples of the divalent metal cation include calcium ion, magnesium ion, zinc ion, manganese ion, ferrous ion, copper ion and the like. Particularly, one or both of calcium ion and magnesium ion (hereinafter to be also referred to as "calcium ion and/or magnesium ion") contributes to the structure formation of a particular compound such as DAG and the like.

The first liquid may contain factors other than a particular compound and a solvent. Examples of the factor include, but are not limited to, physiologically acceptable buffering agent, salt, and isotonic agent.

The first liquid can be prepared by adding a particular compound to the above-mentioned solvent (e.g., water), stirring the mixture at a temperature capable of dissolving the particular compound (e.g., not less than 60°C, not less than 80°C, not less than 90°C), and dissolving until a transparent state is formed. Using DAG subjected to divalent metal cation-exclusion treatment, a dissolution operation is easy since it is dissolved in water without requiring heating. Where necessary, the obtained solution of the particular compound is subjected to a divalent metal cation-exclusion treatment to render the concentration of the divalent metal cation in the solution lower than the structure-forming concentration. Where necessary, a factor other than the particular compound may be added to the solvent in advance, or a factor other than the particular compound may be added to the obtained solution of the particular compound. The first liquid is preferably sterilized. Examples of the method of the sterilization include, but are not limited to, autoclave, filtration sterilization and the like.

[the second liquid]

The second liquid contains a divalent metal cation as a linking substance. Examples of the divalent metal cation include calcium ion, magnesium ion, zinc ion, manganese ion, ferrous ion, copper ion and the like. While the kind of the divalent metal cation is not particularly limited as long as the particular compound in the first liquid is bonded via a divalent metal cation to form a structure capable of suspending cells or tissues, with preference given to calcium ion.

The second liquid is generally a solution of a linking substance (i.e., divalent metal cation). While the solvent for the solution is not particularly limited as long as it can dissolve the particular compound, it is generally water or hydrophilic solvent, preferably water. In a preferable embodiment, therefore, the second liquid is an aqueous solution of a linking substance (i.e., divalent metal cation).

The second liquid contains a divalent metal cation in an amount that renders the concentration of the divalent metal cation in the final liquid medium composition obtained by mixing the first liquid and the second liquid sufficient for the particular compound in the first liquid to form a structure.

The divalent metal cation concentration of the second liquid can be calculated from the divalent metal cation

concentration of the finally obtained liquid medium composition and the mixing ratio of the first liquid and the second liquid.

The second liquid may contain factors other than a linking substance (i.e., divalent metal cation) and the solvent. Examples of the factor include medium constituent components suitable for culturing the intended cells. Examples of the medium constituent component include, but are not limited to, buffering agent (carbonate buffer, phosphate buffer, HEPES etc.), inorganic salts (NaCl etc.), various amino acids, various vitamins (choline, folic acid etc.), saccharides (glucose etc.), antioxidants (monothioglycerol etc.), pyruvic acid, fatty acids, serum, antibiotics, insulin, transferrin, lactoferrin, cholesterol, various cytokines, various hormones, various growth factors, various extracellular matrices, and the like. The second liquid is preferably sterilized. Examples of the sterilization method include, but are not limited to, autoclave, filtration sterilization and the like.

In a preferable embodiment, the second liquid is a concentrate of a liquid medium containing divalent metal cation (preferably, calcium ion and/or magnesium ion) at the concentration allowing formation of a structure. The second liquid contains medium constituent components suitable for culturing the intended cells in addition to divalent metal cation (preferably, calcium ion and/or magnesium ion) and water. The range of the concentrations of calcium ion and magnesium ion in a commonly used liquid medium for culturing cells is about 0.1 - 2.0 mM and 0.1 - 1.0 mM, respectively, and thus they are sufficient to allow DAG and the like to form a structure. The concentration of the divalent metal cation, preferably calcium ion and/or magnesium ion, in the second liquid is adjusted in consideration of the mixing ratio with the first liquid so that the concentration of divalent metal cations in the finally obtained liquid medium composition is the structure formation concentration. The same applies to other linking substances. The concentration of the medium component suitable for culturing the intended cells in the second liquid is adjusted in consideration of the mixing ratio with the first liquid so that the concentration of the medium component in the finally obtained liquid medium composition falls within a concentration range suitable for culturing the intended cells. For example, when the liquid medium composition having a volume of $V_1 + V_2$ is finally obtained by mixing a first liquid having a volume of $V_1$ and a second liquid having a volume of $V_2$, the concentration of the divalent metal cation in the second liquid may be set to $Ci \times (V_1 + V_2)/V_2$ to set the concentration of the divalent metal cation in the liquid medium composition to Ci. The same applies to other linking substances. Similarly, when the first liquid of volume $V_1$ and the second liquid of volume $V_2$ are mixed to finally obtain the liquid medium composition of the volume $V_1 + V_2$, the concentration of the medium constituent component in the second liquid may be set to $Cm \times (V_1 + V_2)/V_2$ to set the concentration of the medium constituent component in the liquid medium composition to Cm.

In this embodiment, the intended liquid medium composition containing a structure in which particular compounds contained in the first liquid are linked via a linking substance contained in the second liquid can be obtained immediately by mixing the first liquid and the second liquid according to the production method of the present invention.

The second liquid may not contain a part or the whole of the medium constituent component for cell culture. In this case, the intended liquid medium composition can be obtained by, in the production method of the present invention, mixing the first liquid and the second liquid to give a mixture containing a structure in which particular compounds contained in the first liquid are linked via a linking substance contained in the second liquid and adding a part or the whole of the above-mentioned liquid culture medium constituent component for cell culture to the mixture.

The volume mixing ratio of the first liquid and the second liquid is 50 to 200, preferably 100, of the volume of the second liquid with respect to the volume 100 of the first liquid.

In a preferable embodiment, 90 mole % or more (preferably 95 mole % or more, more preferably 99% or more, and most preferably 100%) of the particular compound contained in the liquid medium composition produced by the production method of the present invention is derived from the first liquid, and 90 mole % or more (preferably 95 mole % or more, more preferably 99% or more, and most preferably 100%) of the divalent metal cation contained in the medium composition is derived from the second liquid.

[liquid medium composition]

The liquid medium composition that can be obtained by the production method of the present invention contains a structure in which particular compounds contained in the first liquid are linked via a linking substance (i.e., divalent metal cation) contained in the second liquid, wherein the structures are uniformly dispersed in the medium composition. Therefore, using the medium composition, cells and tissues can be cultured while maintaining the suspended state.

The type of organism from which cells or tissues to be cultured are derived is not particularly limited, and may be not only animals (insect, fish, amphibian, reptiles, birds, pancrustacea, hexapoda, mammals and the like) but also plants.

In one embodiment, the cell to be cultured is an anchorage dependent cell. Using the liquid medium composition that can be obtained by the production method of the present invention, anchorage dependent cells can be cultured while maintaining a suspended state, without using a carrier to be the anchorage.

Suspending of cells and/or tissues in the present invention refers to a state where cells and/or tissues may contact the bottom surface but do not adhere to a culture container (non-adhesive). Furthermore, in the present invention,

when the cells and/or tissues are proliferated, differentiated or maintained, the state where the cells and/or tissues are uniformly dispersed and suspended in the liquid medium composition in the absence of a pressure on or vibration of the liquid medium composition from the outside or shaking, rotating operation and the like is referred to as "static suspension", and culturing of the cells and/or tissues in such condition is referred to as "static suspension culture". In the "static suspension", the duration of suspending includes not less than 5 min, not less than 1 hr, not less than 24 hr, not less than 48 hr, not less than 7 days and the like, though the duration is not limited thereto as long as the suspended state is maintained.

The medium composition that can be obtained by the production method of the present invention permits static suspension of cells and/or tissues at least on one point in the temperature range (e.g., 0 - 40°C) capable of maintaining or culturing cells or tissues. The medium composition that can be obtained by the present invention permits static suspension of cells and/or tissues at least at one point in the temperature range of preferably 25 - 37°C, most preferably 37°C.

Whether or not static suspension is possible can be evaluated by, for example, uniformly dispersing the cells to be cultured in a medium composition to be evaluated at a concentration of $2\times10^4$ cells/ml, injecting 10 ml thereof in a 15 ml conical tube, standing the tube for at least not less than 5 min (e.g., not less than 1 hr, not less than 24 hr, not less than 48 hr, not less than 7 days) at a low temperature of about 4°C, and observing whether the suspended state of the cells is maintained. For evaluation of the suspended state, when, for example, not less than 70% of the total cells are in a suspended state, it is concluded that the suspended state was maintained. Polystyrene beads (Size 500-600 μm, manufactured by Polysciences Inc.) may be used for evaluation instead of the cells.

In a preferable embodiment, in the liquid medium composition that can be obtained by the production method of the present invention, the viscosity thereof is not substantially increased by the contained above-mentioned structure. The terms "not substantially increasing the viscosity of the liquid" mean that the viscosity of the liquid does not exceed 8 mPa•s. In this case, the viscosity of the liquid (that is, the viscosity of the liquid medium composition that can be obtained by the production method of the present invention) is not more than 8 mPa•s, preferably not more than 4 mPa.s, more preferably not more than 2 mPa•s at 37°C. The viscosity of the liquid containing the structure can be measured under 37°C conditions and using an E-type viscosity meter (manufactured by Toki Sangyo Co., Ltd., TV-22 type viscosity meter, model: TVE-22 L, corn roter: standard roter 1°34'×R24, rotating speed 100 rpm).

The concentration of the particular compound in the liquid medium composition that can be obtained by the production method of the present invention depends on the kind of the particular compound, and can be appropriately determined within the range where the particular compound can form the aforementioned structure in the liquid medium composition, and can uniformly suspend (preferably statically suspend) the cells and/or tissues (preferably, without substantially increasing the viscosity of the liquid). In the case of DAG, it is 0.001% to 1.0%(w/v), preferably 0.003% to 0.5%(w/v), more preferably 0.005% to 0.3%(w/v), further preferably 0.01% to 0.05%(w/v), most preferably 0.01% to 0.03%(w/v). In the case of xanthan gum, it is 0.001% to 5.0%(w/v), preferably 0.01% to 1.0%(w/v), more preferably 0.05% to 0.5%(w/v), most preferably 0.1% to 0.2%(w/v). In the case of κ-carrageenan and locust bean gum mixture system, the total of the both compounds is 0.001% to 5.00(w/v), preferably 0.005% to 1.0%(w/v), more preferably 0.01% to 0.1%(w/v), most preferably 0.03% to 0.05%(w/v). In the case of a native type gellan gum, it is 0.05% to 1.0%(w/v), preferably 0.05% to 0.1%(w/v).

When plural kinds (preferably two kinds) of the above-mentioned polysaccharides are used in combination as a particular compound, the concentration of the polysaccharides can be appropriately determined within a range where a combination of the polysaccharides can form the aforementioned structure in the liquid medium composition, and the cells and/or tissues can be uniformly suspended (preferably statically suspended) (preferably without substantially increasing the viscosity of the liquid). For example, when a combination of DAG or a salt thereof and polysaccharide other than DAG and a salt thereof is used, the concentration of DAG or a salt thereof is, for example, 0.005 - 0.02%(w/v), preferably 0.01 - 0.02%(w/v), and the concentration of polysaccharide other than DAG and a salt thereof is, for example, 0.0001 - 0.4%(w/v), preferably 0.005 - 0.4%(w/v), more preferably 0.1 - 0.4%(w/v). Specific examples of the combination of the concentration range include the following. DAG or a salt thereof: 0.005 - 0.02% (preferably 0.01 - 0.02%) (w/v)
polysaccharide other than DAG
xanthan gum: 0.1 - 0.4%(w/v)
sodium alginate: 0.0001 - 0.4%(w/v) (preferably 0.1 - 0.4%(w/v))
native gellan gum: 0.0001 - 0.4%(w/v)
locust bean gum: 0.1 - 0.4%(w/v)
methylcellulose: 0.1 - 0.4%(w/v) (preferably 0.2 - 0.4%(w/v))
carageenan: 0.05 - 0.1%(w/v)
diutan gum: 0.05 - 0.1%(w/v)

The concentration can be calculated by the following formula.

$$\text{Concentration [\%(w/v)] = weight (g) of particular compound/cubic volume (mL) of medium composition} \times 100$$

In a preferable embodiment, DAG or a salt thereof is used as the particular compound, and calcium ion is used as the linking substance. The first liquid is an aqueous solution containing DAG or a salt thereof. The second liquid is a concentrate of a liquid medium containing calcium ions. The volume mixing ratio of the first liquid to the second liquid is, as described above, the volume ($V_2$) of the second liquid to the volume ($V_1$) 100 of the first liquid is 50 to 200, preferably 100. The concentration of the DAG in the liquid medium composition obtained as a result of mixing is preferably 0.01% to 0.05% (w/v), most preferably 0.01% to 0.03 (w/v). The concentration of the calcium ion in the liquid medium composition obtained as a result of mixing is the concentration at which the DAG forms a structure, and is usually about 0.1 to 2.0 mM. The concentration of the medium constituent components in the liquid medium composition resulting from mixing is within a concentration range suitable for culturing the intended cells, e.g., mammalian cells.

The DAG concentration in the first liquid is the concentration obtained by multiplying the DAG concentration in the liquid medium composition resulting from the mixing described above by $(V_1 + V_2)/V_1$ (i.e., 150/100 to 300/100, preferably 200/100).

The concentration of the calcium ion in the second liquid is the concentration obtained by multiplying the concentration of the calcium ion in the liquid medium composition obtained as a result of the mixing described above by $(V_1 + V_2)/V_2$ (i.e., 150/50 to 300/200, preferably 200/100).

The concentration of the medium component in the second liquid is the concentration obtained by multiplying the concentration of the medium component in the liquid medium composition obtained as a result of the mixing described above by $(V_1 + V_2)/V_2$ (i.e., 150/50 to 300/200, preferably 200/100).

Since the liquid medium composition contains a structure in which DAG is linked via a calcium ion uniformly dispersed therein, the cells and/or tissues can be uniformly suspended (preferably statically suspended) without substantially increasing the viscosity.

Using the liquid medium composition obtained by the production method of the present invention, cells and/or tissues can be cultured in a suspended state without an operation such as shaking, rotation and the like having a risk of causing damage and loss of functions of cells and tissues. Furthermore, using the medium composition, the medium can be exchanged easily during culture, and the cultured cells and/or tissues can also be recovered easily. Using the medium composition, adhesive cells can be prepared efficiently in a large amount without impairing the function thereof, since cells conventionally required to be cultured on a plate in a single layer in an adhered state to a cell container can be cultured in a suspended state.

The mixture itself of the first liquid and the second liquid may itself be a medium composition for production, or may be a medium composition for production by further adding an additive to the mixture.

[Examples]

By carrying out the production method of the present invention using the production apparatus, the first and second liquids were mixed and the mixed state of the obtained medium composition (the dispersed state of the structure formed by the mixing of the two liquids) was evaluated.

[Specification of each part of production apparatus]

Using the container (flexible bag) 1 shown in Fig. 4 and each tube 41 to 45 and the roller pump 5 as the peristaltic pump, the first and second liquids are mixed to produce the liquid medium composition.

The first liquid is an aqueous solution containing deacylated gellan gum at (concentration: 0.040%), and the second liquid is a liquid medium containing calcium ion at (calcium ion concentration: 3.29 mM).

The container 1 is a bag-like material (bag) using a flexible film made of polyethylene as a material of wall, and its volume is 100 L.

The roller pump is RP-KGI manufactured by Furue Science Co., Ltd. The inner diameter of the circulation tube attached to the roller pump is 13 mm and the outer shape is 19 mm. The feed rate of the roller pump is about 10 L/min. The direction of delivery of the liquid by the roller pump 5 is a direction of the liquid leaving the opening 2, passing the roller pump 5 and reaching the opening 3.

[production of liquid medium composition]

The second liquid (50 L) stored in the outside container 7b was injected into the container 1 in a state of

shrinkage after deflation. Then, the roller pump 5 was operated, and the first liquid (50 L) contained in the external container 7a was injected into the container 1 over 0.5 hr while circulating the second liquid. From the time point when the injection of the first liquid (50 L) was completed, the circulation was continued for 3 hr to complete mixing and the liquid medium composition was obtained.

[Evaluation of mixing state]

Whether or not the liquid medium composition obtained above was preferably mixed, that is, whether or not the structures formed by mixing the first and second liquids (the structure in which the specific compound contained in the first liquid was assembled via the divalent metal cation contained in the second liquid) were in a state of being preferably suspended and dispersed in the liquid medium composition was evaluated by performing the performance evaluation test method (test method newly defined for the present invention) shown below.

[performance evaluation test method]

(1) To sufficiently secure the formation time of the structure, the test is started 48 hours after the completion of mixing of the medium composition.
(2) Polystyrene beads (diameter 600 μm, manufactured by Polysciences) (10 mg) is placed in a 15 mL sterilized conical tube, 10 mL of a medium composition to be evaluated is further injected into the conical tube, and they are mixed by inversion.
(3) Immediately after inversion mixing, uniform dispersing of the beads to the liquid level of the medium composition in the conical tube is visually confirmed.
(4) The sample in the above state (3) is placed in a state in which the conical tube is erected, and the sample is left to stand for 12 hours or more at a temperature of 4°C from the viewpoint of preventing deterioration of the medium components. After the standing, the state of dispersion (whether the beads remain dispersed or tend to settle) is visually judged. When the structure is preferably dispersed and suspended in the medium composition, the beads are also dispersed and remain suspended in the medium composition under the impact of the dispersive suspension of the structure, as schematically shown in FIG. 6(a). On the other hand, when mixing is not preferable and the structure is suspended as a larger aggregate in the medium composition, the remaining portion of the medium composition without the structure also increases. Therefore, as schematically shown in Fig. 6B, the number of beads that sediment without being affected by the suspending of the structure increases. Thus, by mixing the beads in the medium composition, the state of dispersing of the structure, which is difficult to visually confirm, can be confirmed through the beads.
(5) evaluation criteria of dispersion state of beads
Pass: Beads are uniformly dispersed to the liquid surface of the medium composition. In this example, when 10 mL of the medium composition was injected into a 15 mL conical tube, the judgment criteria was that, as schematically shown in Fig. 6(a), the beads were dispersed at or above 9 mL line and collection of sedimented beads was not found at the bottom of the tube.
Failure: The beads are not uniformly dispersed to the liquid surface of the medium composition. In this Example, in contrast to the aforementioned pass criteria, when 10 mL of the medium composition was injected into a 15 mL conical tube, the judgment criteria was that, as schematically shown in Fig. 6(b), no beads were dispersed at or above 9 mL line and collection of sedimented beads was found at the bottom of the tube (especially, lower than 1.5 mL line).

[Evaluation results]

As a result of the above-mentioned performance evaluation test method performed on the medium composition obtained as described above, it was found that the dispersing state of the beads was acceptable, and the production method and the production apparatus of the present invention are excellent method and device capable of preferably mixing the first and second liquids.

[Industrial Applicability]

The production method and the production apparatus of the present invention allow a liquid containing a particular compound to be aseptically, easily and preferably mixed with any liquid containing a linking substance such as a divalent metal cation. As a result, the liquid medium composition in which a fine structure is dispersed can be obtained

inexpensively and in a large amount.

This application is based on a patent application No. 2016-144929 (filing date: July 22, 2016), the contents of which are incorporated in full herein.

[Explanation of Symbols]

| | |
|---|---|
| 1 | container |
| 2, 3 | opening |
| 4 | tube |
| 5 | peristaltic pump |

## Claims

1. A method for producing a liquid medium composition, comprising mixing a first liquid containing a particular compound of the following (i) and a second liquid containing a linking substance of the following (ii), the method comprising

    using a container provided with at least two openings in a wall, wherein said at least two openings are connected to each other by a tube outside the container and wherein the tube has a part with a shape and flexibility that render the tube placeable as a pumping tube into a peristaltic pump; and
    placing the tube into the peristaltic pump, operating the peristaltic pump, and mixing the first liquid and the second liquid by circulating them between the tube and the container to form the liquid medium composition in the container, wherein the liquid medium composition comprises structures formed by said particular compound linked via said linking substance dispersed therein:

        (i) a particular compound which is a polymer compound having an anionic functional group, and capable of forming a structure by linking via a divalent metal cation, which structure being capable of suspending a cell or a tissue,
        (ii) a linking substance which is a divalent metal cation.

2. The production method according to claim 1, wherein

    the particular compound of (i) is deacylated gellan gum, the first liquid is an aqueous solution containing deacylated gellan gum,
    the linking substance of (ii) is a concentrated liquid comprising one or both of calcium ion and magnesium ion, and the second liquid is a liquid culture medium containing one or both of calcium ion and magnesium ion.

3. The production method according to claim 2, wherein a concentration of the deacylated gellan gum in the liquid medium composition is 0.001% (w/v) to 1.0% (w/v).

4. The production method according to any one of claims 1 to 3, wherein a volume mixing ratio of the first liquid and the second liquid is 50 - 200 volume of the second liquid relative to 100 volume of the first liquid.

5. The production method according to any one of claims 1 to 4, wherein said one of the openings is provided as an outlet in a wall constituting a bottom surface of the container or a bottom of a wall constituting a side surface thereof,

    said other opening is provided as an outlet in a wall constituting a top surface of the container or an upper part of the wall constituting the side surface,
    the peristaltic pump is operated, the first liquid and the second liquid move in the tube from the outlet toward the inlet, by which the first liquid and the second liquid in the container are moved in a direction from the top to the bottom.

6. The production method according to any one of claims 1 to 5, wherein said two openings are paired as an outlet and an inlet, the container is provided with a plurality of pairs of the two openings, and the two openings in each pair are connected to each other by a tube, and
    in said second step, each tube is placed into a separate peristaltic pump, the peristaltic pump is operated, and the

first liquid and the second liquid are circulated through each tube.

**7.** The production method according to any one of claims 1 to 6, wherein the container is made of a flexible material so that the volume of the container may vary according to the volume of the liquid contained inside the container, whereby the liquid is injected into the container and discharged to the outside of the container while securing the airtightness in the container.

**8.** The production method according to any one of claims 1 to 7, wherein the container is provided with an opening for an entrance used for putting the liquid into and out from the container,
one of the first liquid and the second liquid is placed in advance in the container, and the other liquid is injected into the container from the opening for an entrance while circulating the liquid by the peristaltic pump.

**9.** A production apparatus used for practicing the production method according to claim 1, comprising

a container for containing the first liquid and the second liquid to be mixed in the production method, wherein at least two openings are provided in a wall of the container, and these openings are connected to each other by a tube outside the container, and
the tube has a part with a shape and flexibility that render the tube placeable into a peristaltic pump configured to deliver a liquid in the tube and to be operable as a pumping tube in the peristaltic pump.

**10.** The production apparatus according to claim 9, wherein said one of the openings is provided in a wall constituting a bottom surface of the container or a bottom of a wall constituting a side surface, and
said other opening is provided in a wall constituting a top surface of the container or an upper part of a wall constituting a side surface.

**11.** The production apparatus according to claim 9 or 10, wherein said two openings are paired as an outlet and an inlet, the container is provided with a plurality of pairs of the two openings, and the two openings in each pair are connected to each other by a tube.

**12.** The production apparatus according to any one of claims 9 to 11, further comprising the peristaltic pump as a constituent element of the production apparatus, wherein the tube is placed in the peristaltic pump and operated as a pumping tube.

**13.** The production apparatus according to claim 12, wherein said one of the openings is provided in a wall constituting a bottom surface of the container or a bottom of a wall constituting a side surface,

said other opening is provided in a wall constituting a top surface of the container or an upper part of a wall constituting a side surface, and
the peristaltic pump is configured to operate such that a liquid in the tube moves from an opening on a lower side to an opening on an upper side, whereby the liquid contained in the container moves downward from above.

**14.** The production apparatus according to any one of claims 9 to 13, wherein the container is made of a flexible material so that the volume of the container may vary according to the volume of the liquid contained inside the container, whereby the liquid is injected into the container and discharged to the outside of the container while securing the airtightness in the container.

**15.** The production apparatus according to any one of claims 9 to 14, wherein the container is further provided with an opening for an entrance used for putting the liquid into and out from the container.

Fig. 1

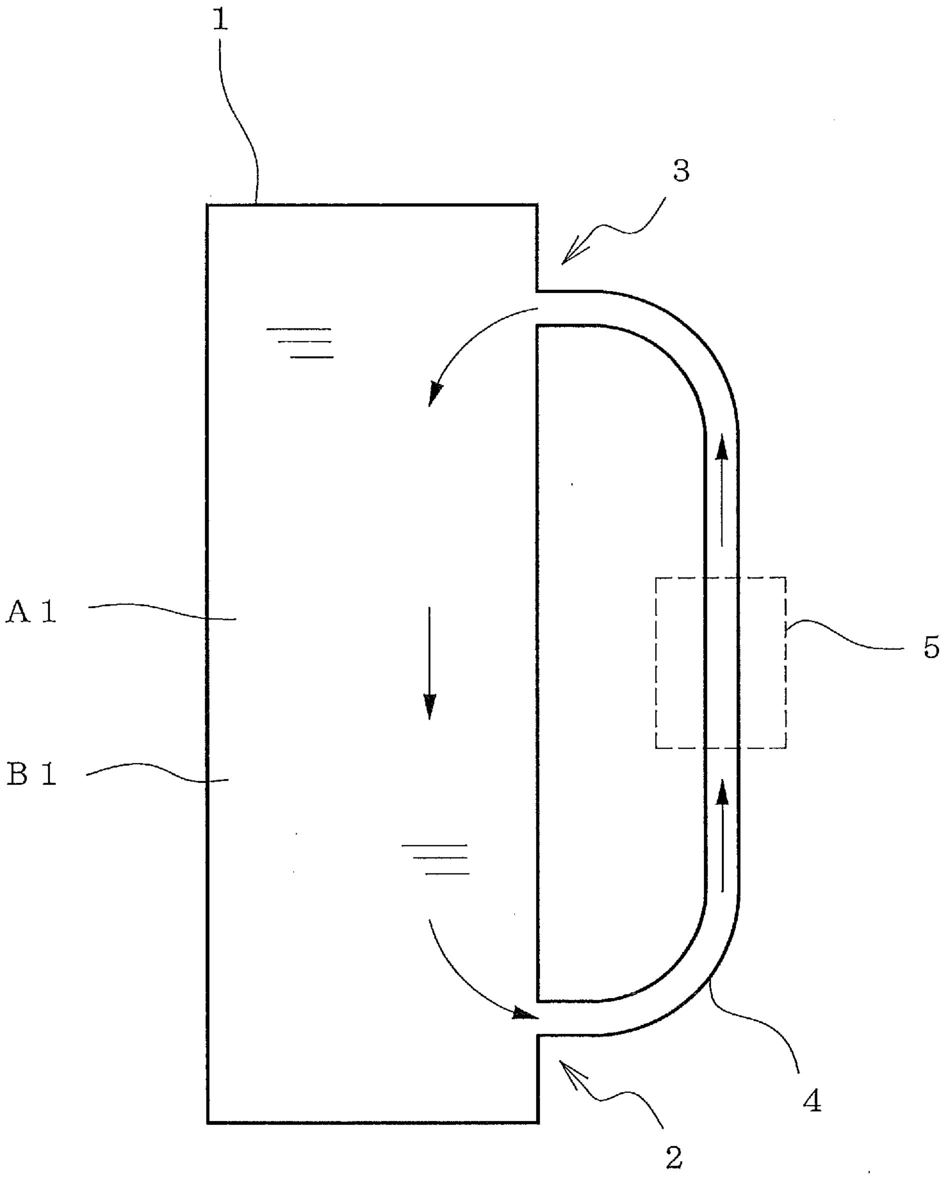

Fig. 2

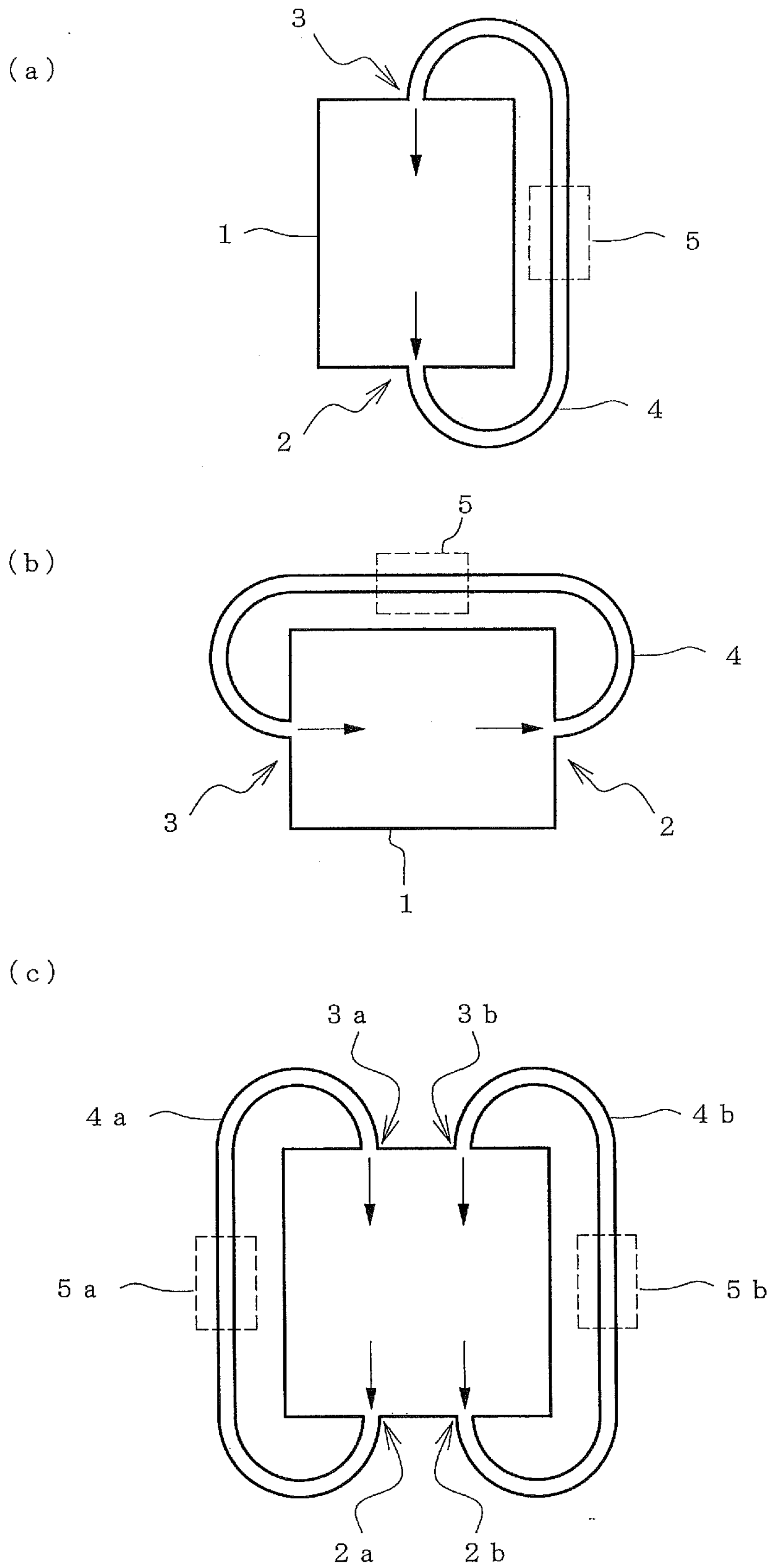

Fig. 3

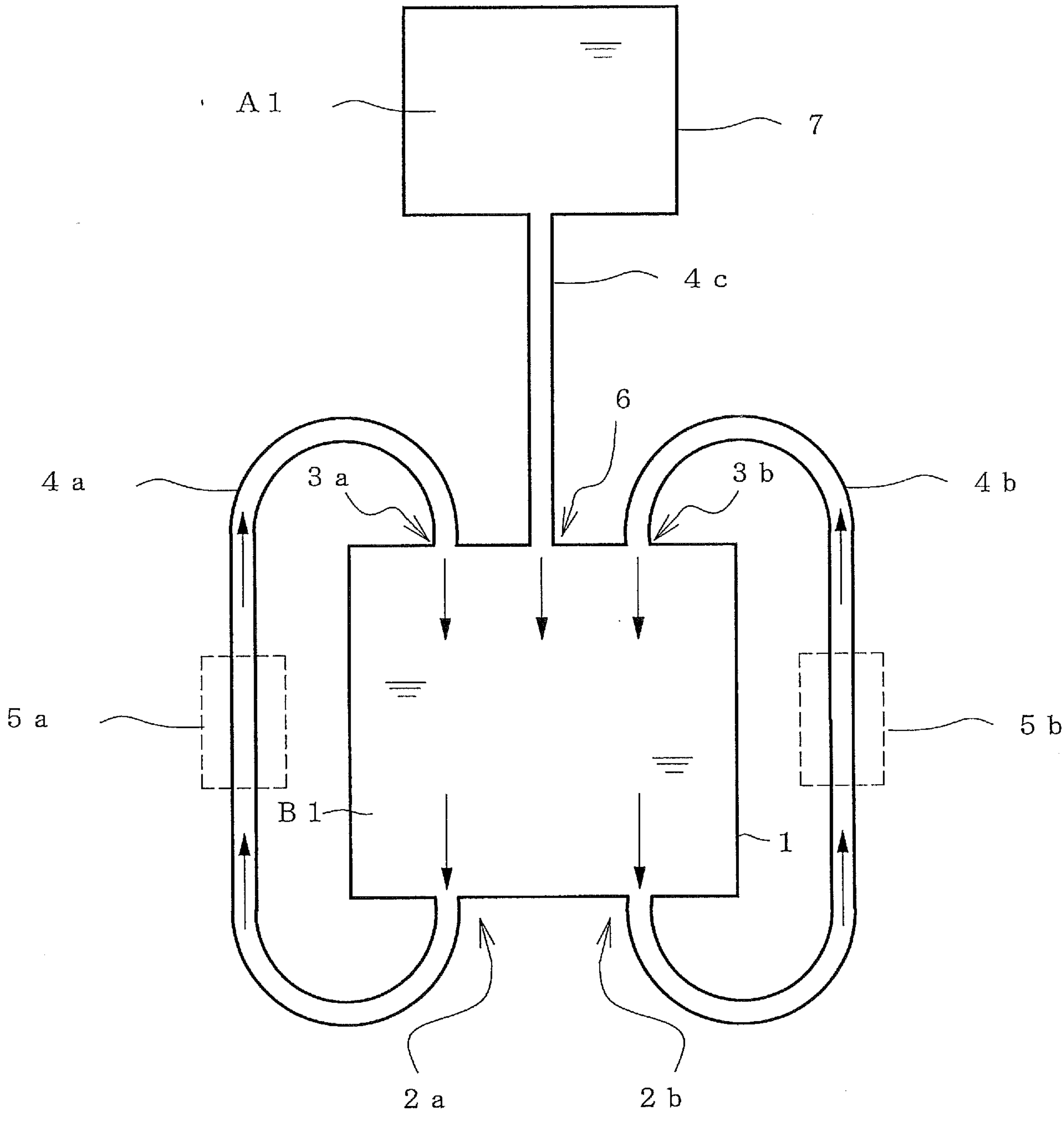

Fig. 4

(a)

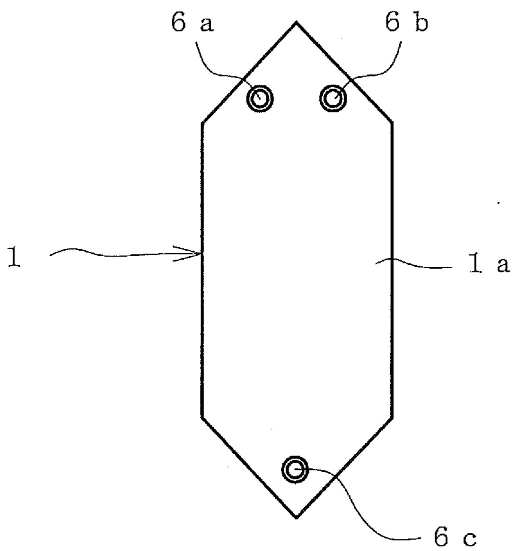
6 a
6 b
1
1 a
6 c

(b)

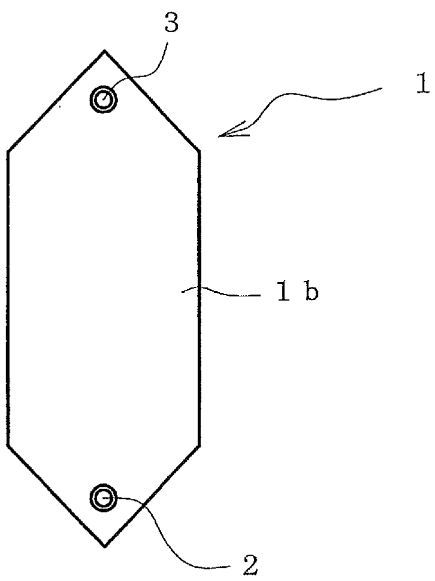
3
1
1 b
2

(c)

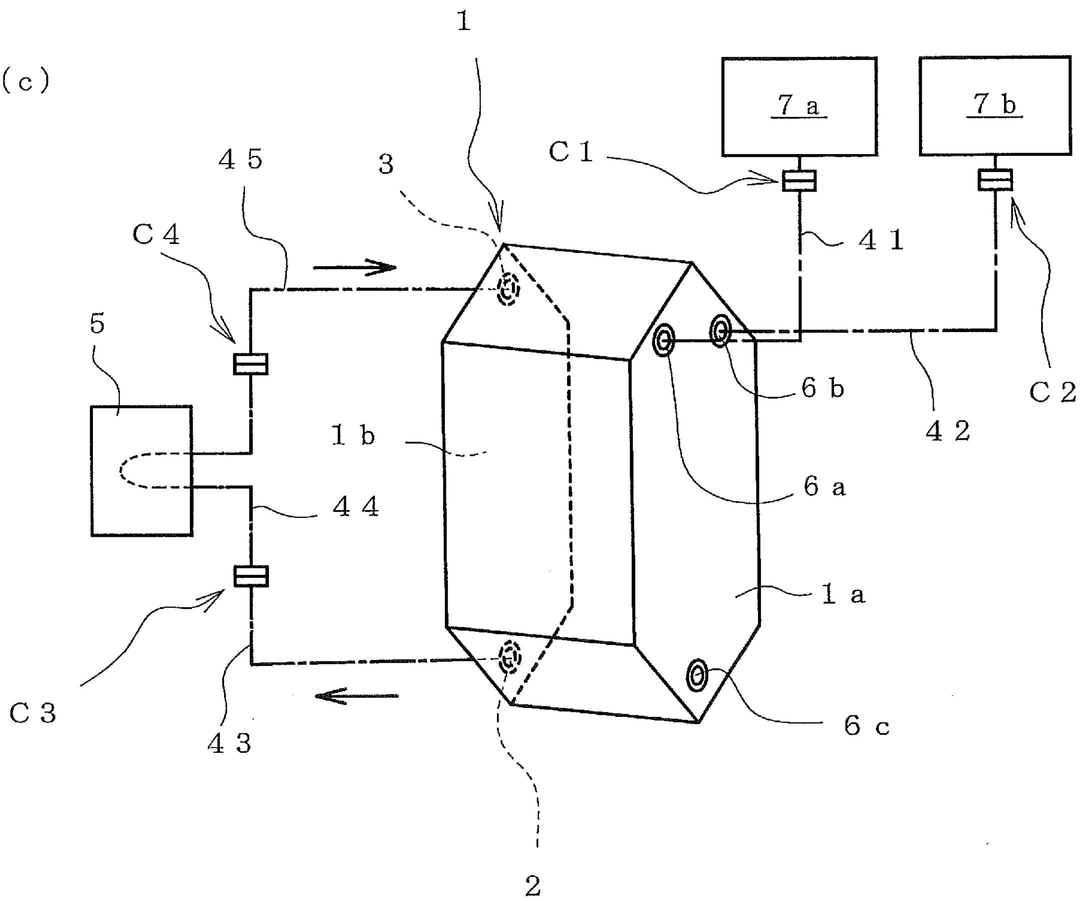
1
7 a
7 b
C 1
4 5
3
C 4
4 1
5
6 b
4 2
C 2
1 b
6 a
4 4
1 a
C 3
4 3
6 c
2

Fig. 5

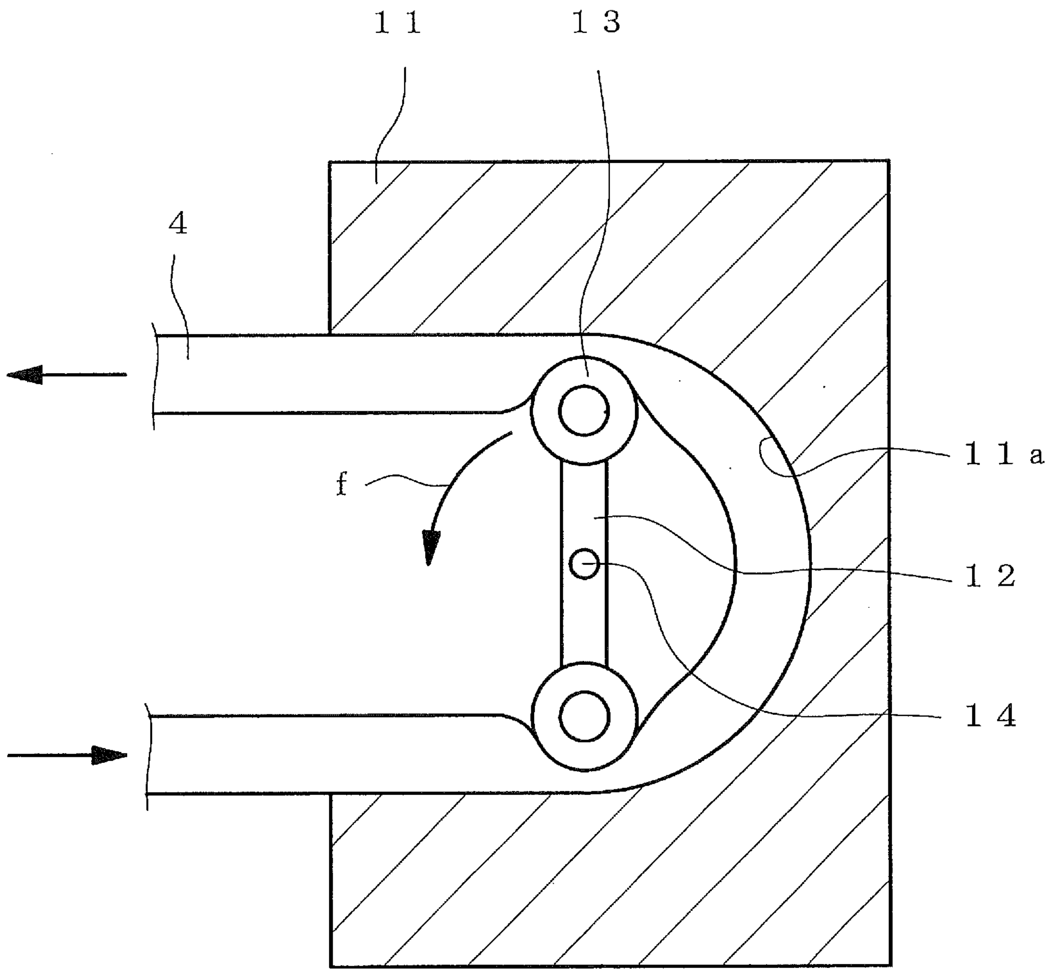

Fig. 6

(a)

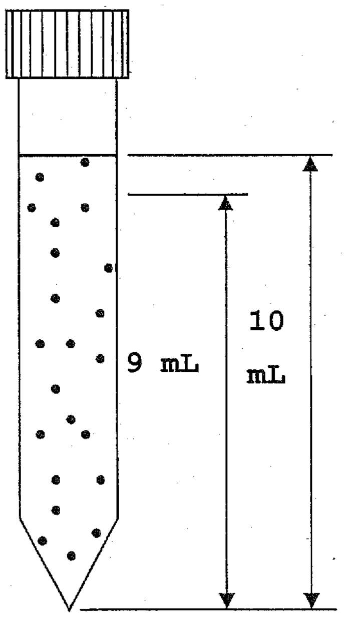


(b)

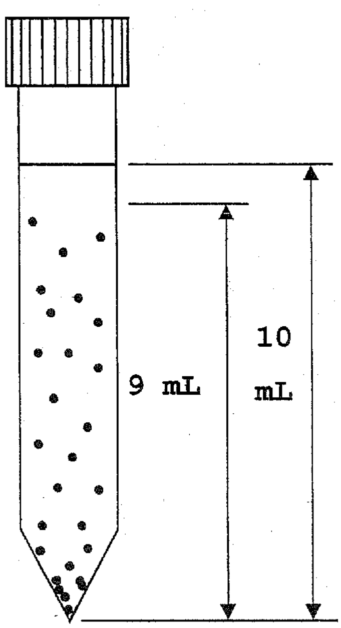

## INTERNATIONAL SEARCH REPORT

International application No. PCT/JP2017/026453

A. CLASSIFICATION OF SUBJECT MATTER
*C12M1/00*(2006.01)i, *C12N1/00*(2006.01)i

According to International Patent Classification (IPC) or to both national classification and IPC

B. FIELDS SEARCHED

Minimum documentation searched (classification system followed by classification symbols)
C12M1/00, C12N1/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN), DWPI(Thomson Innovation)

C. DOCUMENTS CONSIDERED TO BE RELEVANT

| Category* | Citation of document, with indication, where appropriate, of the relevant passages | Relevant to claim No. |
|---|---|---|
| Y | WO 2014/017513 A1 (Nissan Chemical Industries, Ltd.),<br>30 January 2014 (30.01.2014),<br>claims; test example 18<br>& EP 2878664 A1<br>claims; example 18<br>& CA 2879624 A & KR 10-2015-0038190 A<br>& CN 104640974 A | 1-15 |
| Y | WO 2015/111685 A1 (Nissan Chemical Industries, Ltd.),<br>30 July 2015 (30.07.2015),<br>claims<br>& US 2017/000231 A1<br>claims<br>& EP 3098299 A1 & KR 10-2016-0119116 A<br>& CN 106414707 A | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 October 2017 (06.10.17) | 17 October 2017 (17.10.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/026453

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2008-536525 A (Hyclone Laboratories, Inc.), 11 September 2008 (11.09.2008), paragraph [0076] & US 2011/0097789 A1 paragraph [0095] & WO 2006/116069 A1 & EP 1872042 A1 | 1-15 |
| Y | JP 2007-215999 A (Millipore Corp.), 30 August 2007 (30.08.2007), paragraph [0013]; fig. 8 & US 2007/0158360 A1 paragraph [0023]; fig. 8 & EP 1808381 A2 & CN 101032445 A | 1-15 |
| A | JP 2008-519598 A (Agency for Science, Technology and Research), 12 June 2008 (12.06.2008), claims; example 1; fig. 8 & US 2008/0233607 A1 claims; example 2; fig. 1 & EP 1815244 A1 & WO 2006/052223 A1 | 1-15 |
| A | JP 2002-500004 A (Advanced Tissue Sciences, Inc.), 08 January 2002 (08.01.2002), entire text & US 6121042 A whole document & WO 1999/33951 A1 & EP 1042448 A1 | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014017513 A
- US 20140106348 A
- WO 2016144929 A